# EUROPÄISCHE PATENTSCHRIFT

(11) **EP 3 263 627 B1**
(45) Veröffentlichungstag und Bekanntmachung des Hinweises auf die Patenterteilung: **12.08.2020**
(21) Anmeldenummer: 16176762.9
(22) Anmeldetag: 28.06.2016
(51) Int. Cl.: C08J 3/05, C08G 18/08, C08G 18/12, C08G 18/42

(54) **DESTILLATIVE ABTRENNUNG VON KETAZIN AUS POLYURETHANDISPERSIONEN**
DISTILLATIVE SEPARATION OF KETAZIN FROM POLYURETHANE DISPERSIONS
SYSTEME DE SEPARATION PAR DISTILLATION DE CETAZINE A PARTIR DE DISPERSION DE POLYURETHANE

(43) Veröffentlichungstag der Anmeldung: 03.01.2018
(73) Patentinhaber: Covestro Deutschland AG, 51373 Leverkusen (DE)
(72) Erfinder: Grablowitz, Hans Georg, 50733 Köln (DE); Zastrow, Alfred, 41542 Dormagen (DE); Fleck, Olaf, 51467 Bergisch Gladbach (DE)
(74) Vertreter: Levpat

(56) Entgegenhaltungen:
- US-A1- 2007 167 565

## Beschreibung

Die vorliegende Erfindung betrifft ein Verfahren zur Entfernung von Ketazin aus Polyurethandispersionen mittels Destillation unterhalb des Siedepunktes von Ketazin.

Wässrige Polyurethandispersionen (PUDs) spielen aufgrund ihrer besonderen Eigenschaften eine wichtige Rolle bei Beschichtungen. Neben den technischen Vorteilen von Polyurethanen sind wässrige PU-Dispersionen vorteilhaft, da sie nur wenig oder kein organisches Lösungsmittel enthalten und damit einen Beitrag zu nachhaltigen und umweltfreundlichen Beschichtungen liefern. PUDs werden hauptsächlich mittels zwei unterschiedlicher Verfahren hergestellt. Im sog. Schmelz-Dispergierverfahren wird zunächst ein isocyanatfunktionelles, hydrophiles Präpolymer in der Schmelze oder häufig unter Zuhilfenahme eines kleinen Anteils eines zumeist hochsiedenden Lösungsmittels hergestellt. Im nächsten Schritt wird das Präpolymer in Wasser dispergiert und zum Aufbau der Molmasse wird anschließend häufig eine Kettenverlängerung mit Polyaminen oder Polyhydraziden in Wasser durchgeführt. Da das verwendete Lösungsmittel häufig einen hohen Siedepunkt aufweist kann es anschließend nicht entfernt werden und verbleibt in der Dispersion. Im sog. Acetonverfahren wird ebenfalls zunächst ein isocyanatfunktionelles Präpolymer hergestellt welches anschließend in Aceton gelöst wird. Die anschließende Kettenverlängerung erfolgt dann in der acetonischen Lösung des Präpolymeren und die damit verbundene Erhöhung der Viskosität kann über die zugegebene Menge an Aceton kontrolliert werden. Danach erfolgt die Dispergierung mit Wasser und das Aceton kann abschließend destillativ entfernt werden. Da beim Acetonverfahren kein weiteres und häufig hochsiedendes Lösungsmittel eingesetzt wird, sind die resultierenden Dispersionen im Wesentlichen frei von organischen Lösungsmitteln. Bei den Verbindungen zur Kettenverlängerung handelt es sich häufig um Polyamine oder Polyhydrazide und als gängige Verbindung hat sich dabei u.a. Hydrazin bewährt, da die damit umgesetzten Dispersionen eine signifikant höhere Farbstabilität bei thermischer Belastung aufweisen. Setzt man nun Hydrazin zu einer solchen acetonischen Präpolymerlösung hinzu, dann kommt es zur Bildung eines Nebenprodukts indem Aceton und Hydrazin zum Ketazin (oder Acetonazin) reagieren (vgl. auch E.C. Gilbert, Journal of the American Chemical Society, 51, 3394-3409, 1929). Das gebildete Ketazin verbleibt aufgrund seines verhältnismäßig hohen Siedepunkts (134 °C bei Normaldruck) in der Dispersion und kann, je nach Verarbeitungsprozess, wieder zum Hydrazin hydrolysieren, was aufgrund der krebserregenden Wirkung des Hydrazins einen großen Nachteil darstellt.

Aus diesem Grund besteht ein großer Bedarf für ein Verfahren, mit dem das als Nebenprodukt gebildete Ketazin mit geringerem Aufwand und ohne Beeinträchtigung der Qualität der Polyurethandispersion aus der Dispersion entfernt werden kann. Diese Aufgabe wird durch die in den Patentansprüchen und in der Beschreibung charakterisierten Ausführungsformen der vorliegenden Erfindung gelöst.

Als Brüdentemperatur bezeichnet man die Temperatur der Dampfphase, als Sumpftemperatur bezeichnet man die Temperatur der Flüssigphase.

In einer ersten Ausführungsform betrifft die vorliegende Erfindung ein Verfahren zur Entfernung von Ketazin aus einer Polyurethandispersion enthaltend den Schritt der Destillation der Polyurethandispersion, wobei die Brüdentemperatur höchstens 53 °C beträgt, die Destillation bei einem Druck von mindestens 120 mbar und höchstens 140 mbar durchgeführt wird und die Brüdentemperatur 51 °C erreicht.

Die Brüdentemperatur, bei der eine Entfernung des Acetons erreicht wird, kann experimentell sehr einfach bestimmt werden. Der Sumpf der Destillationsanlage wird auf den Siedepunkt von Azeton aufgeheizt. Die Brüdentemperatur wird zunächst unter der Sumpftemperatur liegen, da als erstes die niedrigsiedenden Verbindungen verdampfen. Im Verlauf der Destillation steigt die Brüdentemperatur immer weiter an, da immer höher siedende Verbindungen verdampfen. Durch die Analyse von Proben, welche beim Erreichen definierter Brüdentemperaturen entnommen werden, lässt sich die bei einer gegebenen Brüdentemperatur in der Polyurethandispersion vorliegende Restkonzentration von Aceton einfach bestimmen.

Der Begriff "Entfernung des Acetons" bezeichnet einen Zustand der Polyurethandispersion, bei dem der Acetongehalt geringer als 1 Gew.-% ist.

Es ist weiterhin bevorzugt, dass zusätzlich zu der oben genannten Bedingung die Brüdentemperatur wenigstens um 15 °C unter dem Siedepunkt von Ketazin bei dem in der Destillationsanlage herrschenden Druck liegt. Die vorgenannte Temperaturgrenze wird vorzugsweise nur kurzzeitig, besonders bevorzugt überhaupt nicht, überschritten.

In der Studie, auf der die vorliegende Erfindung basiert, wurde überraschend gefunden, dass das Ketazin destillativ entfernt werden kann, ohne die Temperatur im Sumpf der Destillationsanlage oder im Brüden auf den Siedepunkt von Ketazin anzuheben. Da bei dem in der Anlage herrschenden Druck von 120 mbar das Aceton beim Erreichen einer Brüdentemperatur von 48 °C vollständig entfernt war, während Ketazin unter diesen Bedingungen einen Siedepunkt von ca. 70 °C hat, war es unerwartet, dass eine durch eine Weiterführung der Destillation bis zu einer Brüdentemperatur von 51 °C bereits mehr als 90 % des ursprünglich vorhandenen Ketazins entfernt werden konnte.

Der Begriff "Entfernung des Ketazins" bezieht sich auf einen Zustand der Polyurethandispersion, bei dem die Restkonzentration von Ketazin vorzugsweise weniger als 1000 ppm, stärker bevorzugt weniger als 500 ppm, noch stärker bevorzugt weniger als 300 ppm, noch stärker bevorzugt weniger als 200 ppm und am stärksten bevorzugt weniger als 100 ppm beträgt. Weiterhin ist bevorzugt, dass wenigstens 90 % des ursprünglich in der Polyurethandispersion vorliegenden Ketazins durch das erfindungsgemäße Verfahren entfernt werden.

Dem Fachmann ist die Abhängigkeit des Siedepunktes einer Flüssigkeit vom Umgebungsdruck gut bekannt, so dass er ohne weiteres in der Lage ist, die oben definierten Temperaturgrenzen für verschiedene Drücke in der Destillationsanlage zu bestimmen. Tabelle 1 führt die Siedepunkte von Ketazin für einige Drücke exemplarisch auf.

**Tabelle 1:**

| Druck [mbar] | Siedepunkt Ketazin [°C] |
|---|---|
| 1000 | 133 |
| 500 | 107 |
| 250 | 85 |
| 125 | 69 |

In einer besonders bevorzugten Ausführungsform der vorliegenden Erfindung wird die Destillation bei einem Druck von höchstens 140 mbar, besonders bevorzugt höchstens 125 mbar, durchgeführt. Noch stärker bevorzugt wird die Destillation bei einem Druck zwischen 115 mbar und 125 mbar durchgeführt. Hierbei wird eine Brüdentemperatur von 53 °C nicht dauerhaft überschritten. Stärker bevorzugt wird die Destillation bei einem Druck zwischen 115 mbar und 125 mbar durchgeführt.

Der Fachmann weiß, dass der Druck in der Destillationsanlage nicht während des ganzen Destillationsprozesses in den oben angegebenen Grenzen liegen muss. Der Destillationsprozess kann erfindungsgemäß bei Umgebungsdruck begonnen werden und dann bei kontinuierlich oder schrittweise abgesenktem Druck weitergeführt werden. In diesem Zusammenhang sind kurzzeitige Druckanstiege unschädlich, wenn sie den Destillationsprozess nicht beeinträchtigen.

In einer bevorzugten Ausführungsform der vorliegenden Erfindung wird hierbei eine Sumpftemperatur von 53 °C nicht dauerhaft überschritten. Die Destillation wird vorzugsweise bei einer Sumpftemperatur von 45 °C begonnen.

Eine "dauerhafte" Überschreitung einer Temperaturgrenze, wie in der vorliegenden Patentanmeldung verstanden, bedeutet, dass während des gesamten Destillationsvorgangs die angegebene Temperaturgrenze für höchstens 15 Minuten, stärker bevorzugt höchstens 10 Minuten und am stärksten bevorzugt höchstens 5 Minuten überschritten wird. Spiegelbildlich bedeutet eine "nur kurzeitige" Überschreitung einer Temperaturgrenze, dass besagte Temperaturgrenze während des gesamten Destillationsvorgangs für höchstens 15 Minuten, stärker bevorzugt höchstens 10 Minuten und am stärksten bevorzugt höchstens 5 Minuten überschritten wird. Die vorgenannten Zeiträume beziehen sich auf die Gesamtdauer der kontinuierlichen oder in wenigstens zwei getrennte Phasen geteilten Temperaturüberschreitung.

In einer besonders bevorzugten Ausführungsform der vorliegenden Erfindung wird die Destillation nach Erreichen einer Brüdentemperatur, bei der eine Entfernung des Acetons erreicht ist, bevorzugt für wenigstens 120 Minuten, stärker bevorzugt für 30 Minuten bis 180 Minuten, noch stärker bevorzugt für 60 Minuten bis 150 Minuten, und am stärksten bevorzugt für 90 Minuten bis 150 Minuten fortgesetzt.

Die Polyurethandispersion, aus der das Ketazin mit dem erfindungsgemäßen Verfahren entfernt wird, wird vorzugsweise nach dem in der Einleitung beschriebenen Acetonverfahren hergestellt.

Die nach dem erfindungsgemäßen Verfahren zu reinigenden Polyurethandispersionen werden aus den für die Herstellung von Polyurethandispersionen geeigneten Präpolymeren durch Kettenverlängerung hergestellt.

Bei den für die Erfindung geeigneten Präpolymeren handelt es sich typischerweise um die Reaktionsprodukte eines oder mehrerer Polyisocyanate mit einem oder mehreren isocyanatreaktiven Verbindungen, wobei das Polyisocyanat oder die Polyisocyanate im stöchiometrischen Überschuss eingesetzt wird so dass das Präpolymer endständige Isocyanatgruppen aufweist.

Die geeigneten Präpolymere können weiterhin in hydrophobe und hydrophile Präpolymere unterschieden werden. Als hydrophobe Präpolymere werden solche Verbindungen bezeichnet, die keine hydrophilen Gruppen aufweisen und somit nicht in Wasser gelöst oder dispergiert werden können. Als hydrophile Präpolymere werden solche Verbindungen bezeichnet, die kovalent gebundene hydrophile Gruppen aufweisen, die es ermöglichen, das Präpolymer in Wasser zu lösen oder zu dispergieren.

Geeignete Polyisocyanate sind aromatische, araliphatische, aliphatische oder cycloaliphatische Polyisocyanate. Es können auch Mischungen solcher Polyisocyanate eingesetzt werden. Beispiele geeigneter Polyisocyanate sind Butylendiisocyanat, Hexamethylendiisocyanat (HDI), 1,5-Pentamethylendiisocyanat, Isophorondiisocyanat (IPDI), 2,2,4 und/oder 2,4,4-Trimethyl¬hexa¬methy¬len¬-diiso¬cyanat, die isomeren Bis(4,4'-isocyanatocyclo¬hexyl)¬methane oder deren Mischungen beliebigen Isomerengehalts, Isocyanato¬methyl-1,8-octandiisocyanat, 1,4-Cyclohexylendiisocyanat, 1,4-Phenylendiisocyanat, 2,4- und/oder 2,6-Toluylendiisocyanat, 1,5-Naphthylen¬diisocyanat, 2,4'- oder 4,4'-Diphenylmethandiisocyanat, Triphenylmethan-4,4',4"-triisocyanat oder deren Derivate mit Urethan-, Isocyanurat-, Allophanat-, Biuret-, Uretdion-, Iminooxadiazindionstruktur und Mischungen derselben. Bevorzugt sind Hexamethylendiisocyanat, Isophorondiisocyanat und die isomeren Bis(4,4'-iso¬cyanato-cyclohexyl)methane sowie deren Mischungen.

Als isocyanatreaktives Polyol im Sinne der vorliegenden Erfindung wird eine Verbindung bezeichnet die 1 - 4 isocyanatreaktive Gruppen aufweist, bevorzugt 1,5 - 2,5 und ganz besonders bevorzugt 1,9 - 2,1. Als isocyanatreaktive Gruppen eignen sich die dem Fachmann bekannten Gruppen wie beispielsweise Hydroxygruppen, Amingruppen, Hydrazidgruppen oder Thiolgruppen, bevorzugt Hydroxygruppen oder Amingruppen, ganz besonders bevorzugt Hydroxygruppen.

Die Molmasse der geeigneten Polyole liegt zwischen 40 g/mol - 13000 g/mol und als Polyole eignen sich niedermolekulare, diskrete Verbindungen und/oder höhermolekulare, polydisperse Verbindungen. Bei den niedermolekularen Verbindungen handelt es sich üblicherweise um diskrete Verbindungen im Molmassenbereich zwischen 40 und 499 g/mol. Bei den höhermolekularen Verbindungen handelt es sich um Verbindungen die eine Molekulargewichtsverteilung aufweisen und deren mittleres zahlengemitteltes Molgewicht zwischen 500 und 13000 liegt, bevorzugt zwischen 700 g/mol und 4000 g/mol, ganz besonders bevorzugt zwischen 1000 g/mol und 3000 g/mol.

Geeignete niedermolekulare Polyole sind kurzkettige, d.h. 2 bis 20 Kohlenstoff¬atome enthaltende aliphatische, araliphatische oder cycloaliphatische Verbindugnen. Beispiele für Diole sind Ethy¬l¬englykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethyl-pentandiol, stellungsisomere Diethyloctandiole, 1,3-Bu¬tylen¬glykol, Cyclohexandiol, 1,4-Cyclohexandimethanol, 1,6-Hexandiol, 1,2- und 1,4-Cyclohexandiol, hydriertes Bisphenol A (2,2-Bis(4-hy¬droxy¬cyclohexyl)propan), 2,2-Dimethyl-3-hydroxy¬propion¬säure-(2,2-dimethyl-3-hydroxypropylester). Bevorzugt sind 1,4-Butandiol, 1,4-Cyclohexandimethanol und 1,6-Hexandiol. Beispiele geeigneter Triole sind Trimethylolethan, Trimethylolpropan oder Glycerin, bevorzugt ist Trimethylol-propan.

Beispiele für Diamine sind 1,2-Ethylendiamin, 1,6-Hexamethylendiamin, 1,4-Butandiamin und Isophorondiamin, besonders bevorzugt sind 1,2-Ethylendiamin und Isophorondiamin. Beispiele für Dihydrazide sind Oxalsäuredihydrazid, Carbohydrazid und Adipinsäuredihydrazid, besonders bevorzugt sind Carbohydrazid und Adipinsäuredihydrazid. Beispiele für Dithiole sind 1,2-Ethandithiol, 1,3-Propandithiol, 1,4-Butandithiol und 1,6-Hexandithiol. Besodners bevorzugt sind 1,2-Ethandithiol und 1,6-Hexandithiol.

Als niedermolekulare Verbindungen werden bevorzugt Diole verwendet.

Bei den höhermolekularen Verbindungen handelt es sich um Verbindungen die ihrerseits aus Monomeren aufgebaut sind und die neben den meist terminalen Isocyanat-reaktiven Endgruppen weitere funktionelle Gruppen entlang der Hauptkette aufweisen.

Geeignete höhermolekulare Polyole sind Poly¬ester¬poly¬ole, Poly¬acrylatpolyole, Poly¬urethan¬poly¬ole, Polycarbonat¬polyole, Poly¬ether¬polyole, Polyester¬poly¬¬acrylat¬polyole, Poly¬urethan¬poly¬acrylat¬polyole, Poly¬urethan¬poly¬ester¬polyole, Poly¬urethan¬poly¬etherpolyole, Poly¬urethan¬poly¬carbonat¬polyole und Polyester¬poly¬car¬bo¬nat¬polyole, Polyetherpolyamine und Polyamidopolyamine besonders bevorzugt sind Polyesterpolyole, Polyetherpolyole und Polycarbonatpolyole, besonders bevorzugt sind Polyesterpolyole.

Die geeigneten Polyesterpolyole sind häufig aus ein oder mehreren aliphatischen und/oder aromatischen und/oder araliphatischen Dicarbonsäuren mit ein oder mehreren aliphatischen und/oder aromatischen und/oder araliphatischen Diolen aufgebaut und werden über einen Polykondensationsprozess hergestellt.

Gut geeignete Beispiele für Polyesterpolyole sind die bekannten Polykondensate aus Di- sowie gegebenenfalls Tri,- und Tetraolen und Di- sowie gegebenenfalls Tri- und Tetra)carbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden. Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopenthylglykolester, wobei die drei letztgenannten Verbindungen bevorzugt sind. Um eine Funktionalität < 2 zu erzielen können gegebenenfalls Polyole mit einer Funktionalität von 3 anteilig verwendet werden, beispiels-weise Trimethylolpropan, Glycerin, Erythrit, Pentaerythrit, Triemthylolbenzol oder Trishydroxyethylisocyanurat zu nennen.

Als Dicarbonsäuren kommen beispielsweise in Frage Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, Bernsteinsäure, 3,3-Diethylglutarsäure, 2,2-Dimethylbernsteinsäure. Anhydride dieser Säuren sind ebenfalls brauchbar, soweit sie existieren. Für die Belange der vorliegenden Erfindung werden die Anhydride infolgedessen durch den Ausdruck "Säure" umfasst. Es können auch Monocarbonsäuren, wie Benzoesäure und Hexancarbonsäure verwendet werden, vorausgesetzt, dass die mittlere Funktionalität des Polyols 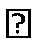 2 ist. Gesättigte aliphatische oder aromatische Säuren sind bevorzugt, wie Adipinsäure oder Isophthalsäure. Als gegebenenfalls in kleineren Mengen mitzuverwendende Polycarbonsäure sei hier Trimellitsäure genannt.

Hydroxycarbonsäuren, die als Reaktionsteilnehmer bei der Herstellung eines Polyesterpolyols mit endständigen Hydroxylgruppen mitverwendet werden können sind beispielsweise Hydroxycapronsäure, Hydroxybuttersäure, Hydroxydecansäure, Hydroxystearinsäure und dergleichen. Brauchbare Lactone sind u.a. ε-Caprolacton, Butyrolacton und Homologe.

Bevorzugt sind Polyesterpolyole b) auf Basis von Butandiol und/oder Neopentylglykol und/oder Hexandiol und/oder Ethylenglykol und/oder Diethylenglykol mit Adipinsäure und/oder Phthalsäure und/oder Isophthalsäure. Besonders bevorzugt sind Polyesterpolyole b) auf Basis von Butandiol und/oder Neopentylglykol und/oder Hexandiol mit Adipinsäure und/oder Phthalsäure.

Als Polyetherpolyole seien z.B. die Polyadditionsprodukte der Styroloxide, des Ethylenoxid, Propylenoxid, Tetrahydrofuran, Butylenoxid, Epichlorhydrins, sowie ihre Mischadditions- und Pfropfprodukte, sowie die durch Kondensation von mehrwertigen Alkoholen oder Mi-schungen derselben und die durch Alkoxylierung von mehrwertigen Alkoholen, Aminen und Aminoalkoholen gewonnenen Polyetherpolyole genannt.

Geeignete hydroxyfunktionelle Polyether weisen OH-Funktionalitäten von 1,5 bis 6,0, bevorzugt 1,8 bis 3,0, OH-Zahlen von 50 bis 700, bevorzugt von 100 bis 600 mg KOH/g Feststoff und Molekulargewichte Mn von 106 bis 4 000 g/mol, bevorzugt von 200 bis 3500 auf, wie z.B. Alkoxylierungsprodukte hydroxyfunktioneller Startermolekuele wie Ethylenglykol, Propylenglykol, Butandiol, Hexandiol, Trimethylolpropan, Glycerin, Pentaerythrit, Sorbit oder Gemische dieser und auch anderer hydroxyfunktioneller Verbindungen mit Propylenoxid oder Butylenoxid. Bevorzugt als Polyetherkomponente b) sind Polypropylenoxidpolyole und Polytetramethylenoxidpolyole mit einem Molekulargewicht von 300 bis 4000 g/mol. Hierbei können die besonders niedermolekularen Polyetherpolyole bei entsprechend hohen OH-Gehalten wasserlöslich sein. Besonders bevorzugt sind jedoch wasserunlösliche Polypropylenoxidpolyole und Polytetramethylenoxidpolyole mit einem Molgewicht von 500 - 3000 g/mol sowie deren Mischungen.

Die in Frage kommenden Polycarbonatpolyole sind durch Reaktion von Kohlensäurederivaten, z.B. Diphenylcarbonat, Dimethylcarbonat oder Phosgen mit Diolen erhältlich. Als derartige Diole kommen z.B. Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutlyenglykol, Polybutylenglykole, Bisphenol A, Tetrabrombisphenol A, aber auch Lacton modifizierte Diole in Frage. Bevorzugt enthält die Diolkomponente 40 bis 100 Gew.-% 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol ε-Caprolacton oder durch Veretherung von Hexandiol mit sich selbst zum Di- oder Trihexylenglykol erhalten werden. Auch Polyether-Polycarbonatpolyole können eingesetzt werden.

Bevorzugt sind Polycarbonatpolyole b) auf Basis von Dimethylcarbonat und Hexandiol und/oder Butandiol und/oder ε-Caprolacton. Ganz besonders bevorzugt sind Polycarbonatpolyole auf Basis von Dimethylcarbonat und Hexandiol und/oder ε-Caprolacton.

Die hydrophilen Präpolymere enthalten weiterhin ionische Gruppen und/oder nicht-ionische, hydrophile Gruppen, um eine ausreichende Dispergierung der resultierenden Polyurethandispersion in Wasser zu gewährleisten. Die Ionischen Gruppen können dabei entweder kationischer oder anionischer Natur sein. Kationisch, anionisch oder nichtionisch dispergierend wirkende Verbindungen sind solche, die beispielsweise Sulfonium-, Ammonium-, Phosphonium-, Carboxylat-, Sulfonat-, Phosphonat-Gruppen oder die Gruppen, die durch Salzbildung in die vorgenannten Gruppen überführt werden können (potentiell ionische Gruppen) oder Polyethergruppen enthalten und durch vorhandene isocyanatreaktive Gruppen in die Makromoleküle eingebaut werden können. Die zur Salzbildung notwendigen Neutralisationsmittel können im Verhältnis zur salzbildenden Gruppe entweder stöchiometrisch oder im Unterschuss hinzu gegeben werden. Zur Erzeugung von anionischen Gruppen werden organische Basen , wie tertiäre Amine, oder anorganische Basen, wie Alkalimetallhydroxide oder Ammoniak hinzu gegeben. Bevorzugt werden dabei tertiäre Amine wie das Triethylamin, Triethanolamin oder Dimethylethanolamin eingesetzt. Bevorzugte geeignete isocyanatreaktive Gruppen sind Hydroxyl- und Amingruppen.

Geeignete ionische oder poten¬tiell ionische Verbindungen sind z.B. Mono- und Dihydroxy¬carbonsäuren, Dihydrohxydicarbonsäuren, Mono- und Diaminocarbonsäuren, Mono- und Dihydroxy¬sulfonsäuren, Mono- und Diaminosulfonsäuren sowie Mono- und Dihydroxy¬phos¬phon¬säuren oder Mono- und Diaminophosphonsäuren und ihre Salze wie Dimethylol¬propionsäure, Dimethylolbuttersäure, Hydroxy¬pivalinsäure, N-(2-Amino¬ethyl) -alanin, 2-(2-Amino-ethylamino)-ethan¬sulfon¬säure, Ethylen-diamin-propyl- oder butylsulfonsäure, 1,2- oder 1,3-Propylen¬diaminethyl¬sulfonsäure, Äpfelsäure, Zitronen¬säure, Glykolsäure, Milchsäure, Glycin, Alanin, Taurin, Lysin, 3,5-Diamino¬benzoesäure, ein Additionsprodukt von IPDI und Acrylsäure (EP-A 0 916 647, Beispiel 1) und dessen Alkali- und/oder Ammonium¬salze; das Addukt von Natrium¬bisulfit an Buten-2-diol-1,4, Polyether¬sulfonat, das propoxylierte Addukt aus 2-Butendiol und NaHSO3, z.B. beschrieben in der DE-A 2 446 440 (Seite 5-9, Formel I-III) sowie in kationische Gruppen überführbare Bau¬steine wie N-Methyl-diethanol¬amin als hydrophile Aufbaukomponenten. Bevorzugte ionische oder poten¬tielle ionische Verbindungen sind solche, die über Carboxy- oder Carboxylat- und/oder Sulfonatgruppen und/oder Ammonium¬gruppen verfügen.

Bevorzugte Verbindungen sind Polyethersulfonat, Dimethlyolpropionsäure, Weinsäure und Dimethylolbuttersäure, besonders bevorzugt sind Polyethersulfonat und Dimethylolpropionsäure.

Geeignete nichtionisch hydrophilierend wirkende Verbindungen sind z.B. Poly¬oxyalkylenether, die mindestens eine Hydroxy- oder Aminogruppe enthalten. Diese Polyether enthalten einen Anteil von 30 Gew.-% bis 100 Gew.-% an Bausteinen, die vom Ethylenoxid abgeleitet sind. In Frage kommen linear aufgebaute Polyether einer Funktionalität zwischen 1 und 3, aber auch Verbindungen der allgemeinen Formel (I), 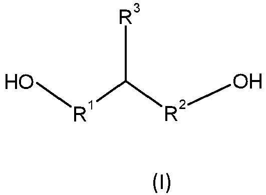 in welcher
R1 und R2 unabhängig voneinander jeweils einen zweiwertigen aliphatischen, cycloaliphatischen oder aromatischen Rest mit 1 bis 18 C-Atomen, die durch Sauerstoff und/oder Stickstoffatome unterbrochen sein können, bedeuten und
R3 für einen alkoxyterminierten Polyethylenoxidrest steht.

Nichtionisch hydrophilierend wirkende Verbindungen sind beispielsweise auch ein¬wertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ull¬manns Encyclopädie der technischen Chemie, 4. Auflage, Band 19, Verlag Chemie, Weinheim S. 31-38).

Geeignete Startermoleküle sind beispielsweise gesättigte Monoalkohole wie Metha-nol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methyl¬cyclohexanole oder Hydroxymethylcyclohexan, 3 Ethyl-3-hydroxymethyloxetan oder Tetrahydrofurfurylalkohol, Diethylenglykol-monoalkylether wie beispielsweise Diethylen¬glykolmonobutylether, ungesättigte Alkohole wie Allylalkohol, 1,1-Di¬methyl-allylalkohol oder Oleinalkohol, aromatische Alkohole wie Phenol, die iso¬meren Kresole oder Methoxyphenole, araliphatische Alkohole wie Benzylalkohol, Anis¬alkohol oder Zimtalkohol, sekundäre Monoamine wie Dimethylamin, Diethyl¬amin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylen-glykolmonobutylether als Startermolekül verwendet.

Für die Alkoxylierungsreaktion geeignete Alkylenoxide sind insbesondere Ethylen¬oxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylen¬oxid¬einheiten zu mindestens 30 mol-% bevorzugt zu mindestens 40 mol-% aus Ethylen¬oxideinheiten bestehen. Bevorzugte nichtionische Verbindungen sind mono¬funk¬tionelle gemischte Polyalkylenoxidpolyether, die mindestens 40 mol-% Ethylen¬oxid- und maximal 60 mol-% Propylenoxideinheiten aufweisen.

Besonders bevorzugt werden monohydroxyfunktionelle Alkoxypolyethylenglykole wie z.B. MPEG 750 (Dow Chemical) und LB 25 (Bayer) und dihydroxyfunktionelle Verbindungen mit lateralen Polyethylenoxid-Einheiten wie z.B. Ymer N 120 (Perstorp) oder Tegomer D 3404.

Das molare Verhältnis von NCO- zu isocyanatreaktiven-Gruppen kann dabei von 1,05 - 4,00 variieren, bevorzugt von 1,2 - 3,0, besonders bevorzugt von 1,4 - 2,5. Die Herstellung der Präpolymere erfolgt indem das entsprechende Polyol oder eine Mischung verschiedener Polyole in einem Reaktionsgefäß vorgelegt wird und anschließend das Polyisocyanat oder die Mischung von Polyisocyanaten bei erhöhter Temperatur zugegeben wird. Wenn Mischungen von Polyolen und/oder Polyisocyanaten verwendet werden, dann können die einzelnen Reaktionspartner auch zu unterschiedlichen Zeitpunkten hinzu gegeben werden um einen gezielten Aufbau des Präpolymeren zu erreichen. Dabei kann die Reaktion entweder in der Schmelze erfolgen oder auch in geeigneten, inerten Lösungsmitteln wie z.B. Aceton oder Butanon. Die Reaktionstemperatur liegt dabei zwischen 50 °C und 130 °C und die Reaktionsdauer beträgt 1 h - 24 h. Die Urethanisierungsreaktion kann durch Verwendung von geeigneten Katalysatoren beschleunigt werden. Dazu eignen sich die dem Fachmann bekannten Katalysatoren wie z. B. Triethyl¬amin, 1,4-Diazabicyclo-[2,2,2]-octan, Zinndioktoat, Dibutylzinndilaurat oder Wismutdioktoat, die mit vorgelegt oder später zudosiert werden. Bevorzugt ist Dibutylzinndilaurat. Die Reaktion ist üblicherweise dann beendet, wenn sich der NCO-Gehalt nicht mehr ändert, eine Reaktionskontrolle erfolgt dabei üblicherweise durch Titration. Um die weitere Verarbeitung des Präpolymeren zu gewährleisten sind generell niedrig viskose Präpolymere von Vorteil, dazu wird, falls nicht während der Herstellung geschehen, das Präpolymer in einem geeigneten Lösungsmittel gelöst. Als niedrig viskose Präpolymere oder der Präpolymer- Lösungen werden solche Systeme bezeichnet deren Viskosität bei einer Scherrate von 40 s-1 < 104 mPas liegt. Die Präpolymerlösung hat dabei bevorzugt einen Feststoffanteil von > 40% und als Lösungsmittel wird Aceton bevorzugt.

Für das erfindungsgemäße Verfahren sind vorzugsweise solche Polyurethandispersionen geeignet, bei denen die Kettenverlängerung durch Hydrazin erfolgt, da hier durch Ketazin als Nebenprodukt entstehen kann.

Die folgenden Beispiele dienen nur dazu, die Erfindung zu illustrieren.

### Beispiel 1: Herstellung einer Rohdispersion

Die Rohdispersion wurde wie folgt hergestellt: In einem Polymerisationsreaktor wurden ein Polyesterpolyol sowie weitere Polyole mit einer Molmasse < 400 g/mol vorgelegt und auf 70 °C erwärmt. Anschließend wurde eine Polyisocyanatmischung hinzu dosiert und die Reaktorinnentemperatur auf 100 °C erhöht. Die Reaktionsmischung wurde so lange bei 100 °C gerührt, bis der theoretische NCO-Wert von 4,47 Gew.-% erreicht war. Danach wurde das entstandene isocyanatfunktionelle Präpolymer auf 60 °C abgekühlt und in Aceton gelöst. Nach vollständiger Lösung in Aceton wurde die Präpolymerlösung in den Destillationsreaktor überführt und bei 40 °C die wässrige Lösung einer Mischung aus dem Natriumsalz der Aminoethylaminoethansulfonsäure mit Hydrazin zur Kettenverlängerung unter Rühren zugeführt und anschließend weitere 15 min. nachgerührt. Abschließend erfolgte die Dispergierung mit Wasser. Die leicht milchige Polyurethan-Rohdispersion wies einen pH-Wert von 6,9 und einen Feststoffgehalt von 23,8 Gew% auf.

### Beispiel 2 (Vergleich): Konventionelle Destillation der Rohdispersion

Die Rohdispersion wurde im Destillationsreaktor auf 40 °C aufgeheizt. Anschließend wurde ein Vakuum angelegt, das schrittweise auf 120 mbar abgesenkt wurde. Nach Erreichen von 120 mbar stieg die Sumpftemperatur ebenso wie die Brüdentemperatur kontinuierlich an. Beim Erreichen einer Brüdentemperatur von 48,5 °C lag der Acetongehalt der Dispersion unter 1 Gew.-% und die Destillation wurde beendet.

Der Gehalt an Ketazin lag am Destillationsende bei 1100 ppm.

### Beispiel 3: Erfindungsgemäßes Destillationsverfahren

Die Rohdispersion wurde im Destillationsreaktor auf 40 °C aufgeheizt. Anschließend wurde ein Vakuum angelegt, das schrittweise auf 120 mbar abgesenkt wurde. Nach Erreichen von 120 mbar stieg die Sumpftemperatur ebenso wie die Brüdentemperatur kontinuierlich an.

Nach dem Erreichen einer Brüdentemperatur von 48,5 °C wurde die Sumpftemperatur über einen Zeitraum von zwei Stunden bei unverändertem Druck von 120 mbar auf 51 °C erhöht.

Am Ende des erfindungsgemäßen Destillationsprozesses lag der Gehalt an Ketazin bei 65 ppm.

## Patentansprüche

1. Verfahren zur Entfernung von Ketazin aus einer Polyurethandispersion enthaltend den Schritt der Destillation der Polyurethandispersion, wobei die Brüdentemperatur höchstens 53 °C beträgt, die Destillation bei einem Druck von mindestens 120 mbar und höchstens 140 mbar durchgeführt wird und die Brüdentemperatur 51 °C erreicht.

2. Das Verfahren nach Anspruch 1, wobei die Temperatur im Sumpf höchstens 53 °C beträgt.

3. Das Verfahren nach Anspruch 2, wobei die Destillation bei einer Temperatur im Sumpf von höchstens 45 °C begonnen wird und so lange fortgesetzt wird, bis die Temperatur im Sumpf bei maximal 53 °C liegt.

4. Das Verfahren nach einem der Ansprüche 1 bis 3, wobei die Destillation nach Erreichen einer Temperatur im Sumpf von 48 °C für wenigstens zwei Stunden fortgesetzt wird.

5. Das Verfahren nach einem der Ansprüche 1 bis 4, wobei die Polyurethandispersion Aceton und Wasser enthält.

6. Das Verfahren nach einem der Ansprüche 1 bis 5, wobei das Polyurethan durch Kettenverlängerung eines Präpolymers mit Hydrazin aufgebaut wurde.

7. Das Verfahren nach einem der Ansprüche 1 bis 6, wobei der Ketazingehalt der Polyurethandispersion nach dem Ende der Destillation unter 1000 ppm liegt.

## Claims

1. Process for removing ketazine from a polyurethane dispersion comprising the step of the distillation of the polyurethane dispersion, wherein the vapour temperature is at most 53°C, the distillation is carried out at a pressure of at least 120 mbar and at most 140 mbar and the vapour temperature reaches 51°C.

2. Process according to Claim 1, wherein the temperature at the bottom is at most 53°C.

3. Process according to Claim 2, wherein the distillation is commenced at a temperature at the bottom of at most 45°C and is continued until the temperature at the bottom is at maximum 53°C.

4. Process according to any of Claims 1 to 3, wherein the distillation is continued for at least two hours after reaching a temperature at the bottom of 48°C.

5. Process according to any of Claims 1 to 4, wherein the polyurethane dispersion comprises acetone and water.

6. Process according to any of Claims 1 to 5, wherein the polyurethane was constructed by chain extension of a prepolymer with hydrazine.

7. Process according to any of Claims 1 to 6, wherein the ketazine content of the polyurethane dispersion after the end of the distillation is below 1000 ppm.

## Revendications

1. Procédé pour l'élimination de cétazine d'une dispersion de polyuréthane contenant l'étape de la distillation de la dispersion de polyuréthane, la température des vapeurs étant d'au plus 53 °C, la distillation étant mise en œuvre à une pression d'au moins 120 mbars et d'au plus 140 mbars et la température des vapeurs atteignant 51 °C.

2. Procédé selon la revendication 1, la température dans le fond étant d'au plus 53 °C.

3. Procédé selon la revendication 2, la distillation étant débutée à une température dans le fond d'au plus 45 °C et continuée aussi longtemps que la température dans le fond est de maximum 53 °C.

4. Procédé selon l'une quelconque des revendications 1 à 3, la distillation étant continuée pendant au moins deux heures après l'atteinte d'une température dans le fond de 48 °C.

5. Procédé selon l'une quelconque des revendications 1 à 4, la dispersion de polyuréthane contenant de l'acétone et de l'eau.

6. Procédé selon l'une quelconque des revendications 1 à 5, le polyuréthane ayant été construit par extension de chaîne d'un prépolymère avec de l'hydrazine.

7. Procédé selon l'une quelconque des revendications 1 à 6, la teneur en cétazine de la dispersion de polyuréthane après la fin de la distillation étant inférieure à 1 000 ppm.
